# FASCICULE DE BREVET EUROPEEN

(11) **EP 1 110 930 B1**
(45) Date de publication et mention de la délivrance du brevet: **10.09.2003**
(21) Numéro de dépôt: 00403477.3
(22) Date de dépôt: 11.12.2000
(51) Int. Cl.: C07C 2/32, B01J 31/12, C08F 10/00, C08F 4/69

(54) **Composition catalytique et procédé pour l'oligomérisation de l'ethylene, en particulier en hexene-1**
Katalysatorzusammensetzung und Verfahren zur Oligomerisation von Ethylen, insbesondere zu 1-Hexene
Catalytic composition and process for the oligomerisation of ethylene, to primarily 1-hexene

(30) Priorité: 24.12.1999 FR 9916509
(43) Date de publication de la demande: 27.06.2001
(73) Titulaire: Institut Francais du Petrole, 92852 Rueil-Malmaison Cedex (FR)
(72) Inventeur: Commereuc, Dominique, 92190 Meudon (FR); Drochon, Sébastien, 92500 Rueil Malmaison (FR); Saussine, Lucien, 78290 Croissy sur Seine (FR)

(56) Documents cités:
- EP-A- 0 808 849
- GB-A- 1 309 987

## Description

La présente invention concerne un procédé d'oligomérisation de l'éthylène, en particulier en hexène-1, et la composition catalytique utilisée.

Les procédés de production d'alpha oléfines à partir d'éthylène conduisent en général à un ensemble d'oligomères ayant un nombre de carbone de 4 à 30 et même supérieur à 30 et les oléfines sont ensuite séparées par distillation. Depuis quelques années est apparue une demande croissante en oligomères inférieurs, essentiellement butène-1, hexène-1 et octène-1, qui sont utilisés en particulier comme comonomères avec l'éthylène dans la fabrication du polyéthylène basse densité linéaire.

Il existe peu de catalyseurs conduisant sélectivement à la formation d'un oligomère particulier comme c'est le cas dans la dimérisation de l'éthylène en butène-1 avec un catalyseur à base de titane. Il est cependant connu que des catalyseurs à base de chrome peuvent conduire à la formation d'hexène-1 principalement, avec plus ou moins de polyéthylène, la proportion des butènes et des octènes dans les produits étant très faible (R.M. Manyik, W.E. Walker, T.P. Wilson, J. Catal., 1977, 47, 197 et J.R. Briggs, J. Chem. Soc. , Chem. Commun. 1989, 674 et références citées). Des catalyseurs permettant la trimérisation plus ou moins sélective de l'éthylène ont été revendiqués, par exemple dans US-A-5 198 563, US-A-5 288 823, US-A-5 382 738, EP-A-608 447, EP-A-611 743, EP-A-614 865. Ces catalyseurs sont préparés à partir d'un sel de chrome et d'un amidure métallique, un pyrrolure en particulier. D'autres catalyseurs font intervenir un aluminoxane et un complexe du chrome avec une phosphine chélatante (US-A-5 550 305).

Le brevet FR-B-2 764 524 décrit une composition catalytique obtenue par mélange d'au moins un composé du chrome avec au moins un composé aryloxy d'aluminium et au moins un composé d'hydrocarbylaluminium, qui présente une sélectivité particulière pour la formation de butène-1 et/ou d'hexène-1 par oligomérisation de l'éthylène.

Il a maintenant été trouvé selon la présente invention qu'une composition catalytique obtenue en mélangeant au moins un composé du chrome avec au moins un composé aryloxy d'un élément M choisi dans le groupe formé par le magnésium, le calcium, le strontium et le baryum, et avec au moins un composé d'aluminium hydrocarbyle présente une sélectivité particulière pour la formation d'hexène-1 par oligomérisation de l'éthylène.

Plus précisément, ladite composition catalytique comprend un mélange non supporté :
- d'au moins un composé du chrome pouvant comporter un ou plusieurs anions, identiques ou différents, choisis dans le groupe formé par les halogénures, les carboxylates, les acétylacétonates, les anions alcoxy et aryloxy,
- avec au moins un composé aryloxy d'un élément M choisi dans le groupe formé par le magnésium, le calcium, le strontium et le baryum, de formule générale M(RO)₂₋ₙXₙ dans laquelle RO est un radical aryloxy contenant de 6 à 80 atomes de carbone, X est un halogène ou un radical hydrocarbyle contenant de 1 à 30 atomes de carbone, et n est un nombre entier qui prend l'une des valeurs 0 et 1,
- et avec au moins un composé d'aluminium choisi parmi les composés hydrocarbylaluminium répondant à la formule générale AIR'ₘY₃₋ₘ, dans laquelle R' est un radical hydrocarbyle comprenant de 1 à 6 atomes de carbone, Y est un atome de chlore ou de brome et m est un nombre de 1 à 3 (tris(hydrocarbyl)aluminiums, composés chlorés ou bromés d'hydrocarbylaluminium) et les aluminoxanes.

Le composé du chrome peut être un sel de chrome(II) ou de chrome(III), mais aussi un sel de degré d'oxydation différent pouvant comporter un ou plusieurs anions, identiques ou différents, tels que par exemple des halogénures, des carboxylates, des acétylacétonates, des anions alcoxy ou aryloxy. Les composés du chrome utilisés de préférence dans l'invention sont les composés du chrome(III) car ils sont plus accessibles, mais un composé du chrome(I) ou du chrome(II) peut aussi convenir.

Les composés du chrome choisis peuvent être avantageusement solubilisés en milieu hydrocarboné par complexation avec un composé oxygéné organique tel qu'un éther, un ester ou un composé choisi dans la classe des acétals et des cétals (ces derniers résultant de la condensation d'un aldéhyde ou d'une cétone avec un monoalcool ou un polyalcool), tels que par exemple le di-(éthyl-2-hexyloxy)-2,2-propane.

Le composé aryloxy de l'élément M est choisi dans le groupe formé par les composés aryloxy du magnésium, du calcium, du strontium et du baryum, de formule générale M(RO)₂₋ₙXₙ dans laquelle RO est un radical aryloxy contenant de 6 à 80 atomes de carbone, X est un halogène (chlore ou brome) ou un radical hydrocarbyle contenant de 1 à 30 atomes de carbone, linéaire ou ramifié, par exemple alkyle, cycloalkyle, alkényle, aryle, aralkyle, aryle substitué ou cycloalkyle substitué, de préférence un reste hydrocarbyle de 2 à 10 atomes de carbone, et n est un nombre entier qui prend l'une des valeurs 0 et 1.

Les composés aryloxy de l'élément M préférés comportent un radical aryloxy RO qui a pour formule générale : 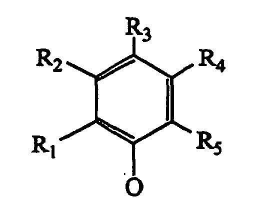 dans laquelle R₁, R₂, R₃, R₄ et R₅, identiques ou différents, représentent chacun un atome d'hydrogène, un atome d'halogène ou un radical hydrocarbyle, par exemple alkyle, cycloalkyle, alkényle, aryle, ou aralkyle, aryle ou cycloalkyle substitué, comprenant de préférence de 1 à 16 atomes de carbone, et plus particulièrement de 1 à 10 atomes de carbone. A titre d'exemples et sans que la liste soit limitative, R₁, R₂, R₃, R₄ et R₅ peuvent être chacun un reste méthyle, éthyle, n-propyle, isopropyle, n-butyle, tert-butyle, cyclohexyle, benzyle, phényle, 2-méthylphényle, 2,6-diméthylphényle, 2,4,6-triméthylphényle ou 2-méthyl-2-phénylprop-1-yle.

Parmi les radicaux aryloxy préférés, on peut citer à titre d'exemples non limitatifs le 4-phénylphénoxy, le 2-phénylphénoxy, le 2,6-diphénylphénoxy, le 2,4,6-triphénylphénoxy, le 2,3,5,6-tétraphénylphénoxy, le 2-tert-butyl-6-phénylphénoxy, le 2,4-ditert-butyl-6-phénylphénoxy, le 2,6-diisopropylphénoxy, le 2,6-diméthylphénoxy,le 2,6-ditert-butylphénoxy, le 4-méthyl-2,6-ditert-butylphénoxy, le 2,6-dichloro-4-tert-butylphénoxy et le 2,6-dibromo-4-tert-butylphénoxy. Lorsque le composé aryloxy de l'élément M est choisi parmi les aryloxydes de formule générale M(RO)_{2,} les deux radicaux aryloxy peuvent être portés par une même molécule, comme par exemple le radical biphénoxy, le binaphtoxy ou le 1,8-naphtalène-dioxy, substitués ou non par un ou plusieurs radicaux alkyle(s), aryle(s) ou halogénure(s).

La préparation du composé M(RO)₂₋ₙXₙ est connue dans la littérature. Tout procédé de préparation de ce composé peut convenir, comme par exemple la réaction d'un phénol ROH avec un élément dialkylmétallique dans un solvant organique, par exemple un hydrocarbure ou un éther.

Les composés d'aluminium utilisés dans l'invention sont choisis parmi les hydrocarbylaluminium -tris(hydrocarbyl)aluminiums, composés chlorés ou bromés d'hydrocarbylaluminium- et les aluminoxanes. Les tris(hydrocarbyl)aluminiums et les composés chlorés ou bromés d'hydrocarbylaluminium sont représentés par la formule générale AIR'ₘY₃₋ₘ dans laquelle R' est un radical hydrocarbyle, de préférence alkyle, comprenant de 1 à 6 atomes de carbone, Y est un atome de chlore ou de brome, de préférence un atome de chlore, et m est un nombre de 1 à 3. On peut citer à titre d'exemples non limitatifs : le dichloroéthylaluminium, le sesquichlorure d'éthylaluminium, le chlorodiéthylaluminium, le chlorodiisobutylaluminium, le triéthylaluminium, le tripropylaluminium, le triisobutylaluminium, le méthylaluminoxane. Le composé d'aluminium hydrocarbyle préféré est le triéthylaluminium.

Les composants du catalyseur peuvent être mis en contact dans un solvant comprenant au moins un hydrocarbure saturé comme l'hexane, le cyclohexane, l'heptane, le butane ou l'isobutane, au moins un hydrocarbure insaturé comme une monooléfine ou une dioléfine comportant par exemple de 4 à 20 atomes de carbone, et/ou au moins un hydrocarbure aromatique tel que le benzène, le toluène, l'ortho-xylène, le mésitylène ou l'éthylbenzène.

La concentration du chrome dans la solution catalytique peut varier de 1.10⁻⁵ à 0,1 mole/L, de préférence de 5.10⁻⁵ à 1.10⁻² mole/L. Le rapport molaire entre le composé aryloxy de l'élément M et le composé du chrome peut varier de 1:1 à 30:1, de préférence de 1:1 à 20:1. Le rapport molaire entre l'aluminium hydrocarbyle et le composé du chrome est choisi entre 1:1 à 35:1, de préférence de 1:1 à 15:1.

L'ordre de mélange des trois constituants de la composition catalytique n'est pas critique. Cependant, on préfère mélanger d'abord le composé du chrome avec le composé aryloxy de l'élément M, et ajouter ensuite le composé d'aluminium hydrocarbyle.

La réaction d'oligomérisation de l'éthylène peut être effectuée sous une pression totale de 0,5 à 15 MPa, de préférence de 1 à 8 MPa, et à une température de 20 à 180 °C, de préférence de 50 à 160 °C.

Dans un mode particulier de mise en oeuvre de la réaction catalytique d'oligomérisation en discontinu, on introduit un volume choisi de la solution catalytique, préparée comme décrit ci-dessus, dans un réacteur muni des dispositifs habituels d'agitation, de chauffage et de refroidissement, puis on pressurise par de l'éthylène à la pression désirée, et on ajuste la température à la valeur souhaitée. Le réacteur d'oligomérisation est maintenu à pression constante par introduction d'éthylène jusqu'à ce que le volume total de liquide produit représente, par exemple, de 2 à 50 fois le volume de la solution catalytique primitivement introduit. On détruit alors le catalyseur par tout moyen habituel connu de l'homme du métier, puis on soutire et on sépare les produits de la réaction et le solvant.

En cas d'opération en continu, la mise en oeuvre est de préférence la suivante : la solution catalytique est injectée en même temps que l'éthylène dans un réacteur agité par les moyens mécaniques classiques ou par une recirculation extérieure, et maintenu à la température souhaitée. On peut aussi injecter séparément les composants du catalyseur dans le milieu réactionnel, par exemple le produit d'interaction du composé du chrome avec le composé aryloxy de l'élément M d'une part, et le composé d'aluminium hydrocarbyle d'autre part. L'éthylène est introduit par une vanne d'admission asservie à la pression, qui maintient celle-ci constante. Le mélange réactionnel est soutiré au moyen d'une vanne asservie au niveau liquide de façon à maintenir celui-ci constant. Le catalyseur est détruit en continu par tout moyen habituel connu de l'homme du métier, puis les produits de la réaction ainsi que le solvant sont séparés, par exemple par distillation. L'éthylène qui n'a pas été transformé peut être recyclé dans le réacteur.

Les exemples suivants illustrent l'invention sans en limiter la portée.

### EXEMPLE 1

Dans un ballon en verre de 100 mL placé sous atmosphère inerte, on introduit à l'abri de l'humidité 0,5.10⁻³ mole d'éthyl-2-hexanoate de chrome(III), dilué avec 25 mL d'ortho-xylène distillé et conservé sous atmosphère inerte.

Dans un autoclave en acier inoxydable d'un volume utile de 100 mL, muni d'une double enveloppe permettant de réguler la température par circulation d'huile, on introduit, dans l'ordre, sous atmosphère d'éthylène et à la température ambiante, 5 mL de la solution d'éthyl-2-hexanoate de chrome(lll) préparée ci-dessus, soit 0,1.10⁻³ mole de chrome, 0,1.10⁻³ mole de bis(diphényl-2,6-phénoxy)-magnésium en solution dans l'ortho-xylène et 0,3.10-3 mole de triéthylaluminium en solution dans l'ortho-xylène. La température est alors portée à 140 °C et la pression d'éthylène est maintenue à 3 MPa.

Après 30 minutes de réaction, l'introduction d'éthylène est arrêtée et le réacteur est refroidi et dégazé, puis le gaz et le liquide, qui a été soutiré au moyen d'une seringue, sont analysés par chromatographie en phase vapeur. On a consommé 19 g d'éthylène en 30 minutes. La composition des produits est donnée dans le Tableau 1. On recueille en outre 11 % en poids de polymère par rapport à l'éthylène consommé.

### EXEMPLE 2

Dans le même appareillage que celui utilisé pour l'Exemple 1 et dans les mêmes conditions, à ceci près que l'on a introduit du bis(t-butyl-4-diphényl-2,6-phénoxy)-magnésium en lieu et place du bis(diphényl-2,6-phénoxy)-magnésium, on a consommé 5,8 g d'éthylène en une heure de réaction. La composition des produits est donnée dans le Tableau 1. On recueille en outre 12,8 % en poids de polymère par rapport à l'éthylène consommé.

### EXEMPLE 3

Dans le même appareillage que celui utilisé pour l'Exemple 1 et dans les mêmes conditions, à ceci près que l'on a introduit 0,2.10⁻³ mole de bis(diphényl-2,6-phénoxy)-magnésium en solution dans l'ortho-xylène, et 0,3.10⁻³ mole de triéthylaluminium en solution dans l'ortho-xylène, on a consommé 18,1 g d'éthylène en 30 minutes de réaction. La composition des produits est donnée dans le Tableau 1. On recueille en outre 22,1 % en poids de polymère par rapport à l'éthylène consommé.

### EXEMPLE 4 (comparatif)

Dans le même appareillage que celui utilisé pour l'Exemple 1 et dans les mêmes conditions, à ceci près que l'on a omis d'introduire le composé du magnésium, on a consommé 1 g d'éthylène en une heure de réaction. La composition des produits est donnée dans le Tableau 1. On recueille en outre 72,5 % en poids de polymère par rapport à l'éthylène consommé.

### EXEMPLE 5

Dans le même appareillage que celui utilisé pour l'Exemple 1 et dans les mêmes conditions, à ceci près que l'on a introduit du bis(phényl-2-t-butyl-6-phénoxy)-magnésium en lieu et place du bis(diphényl-2,6-phénoxy)-magnésium, on a consommé 13,9 g d'éthylène en une heure de réaction. La composition des produits est donnée dans le Tableau 1. On recueille en outre 10,9 % en poids de polymère par rapport à l'éthylène consommé.

### EXEMPLE 6

Dans le même appareillage que celui utilisé pour l'Exemple 1 et dans les mêmes conditions, à ceci près que l'on a introduit du bis(di-t-butyl-2,6-phénoxy)-magnésium en lieu et place du bis(diphényl-2,6-phénoxy)-magnésium, on a consommé 5,4 g d'éthylène en une heure de réaction. La composition des produits est donnée dans le Tableau 1. On recueille en-outre 20,6 % en poids de polymère par rapport à l'éthylène consommé.

### EXEMPLE 7

Dans le même appareillage que celui utilisé pour l'Exemple 1 et dans les mêmes conditions, à ceci près que l'on a introduit 0,2.10⁻³ mole de bis(di-t-butyl-2,4-phényl-6-phénoxy)-magnésium en solution dans l'ortho-xylène, et 0,5.10⁻³ mole de triéthylaluminium en solution dans l'ortho-xylène, on a consommé 19,5 g d'éthylène en 30 minutes de réaction. La composition des produits est donnée dans le Tableau 1. On recueille en outre 22,7 % en poids de polymère par rapport à l'éthylène consommé.

### EXEMPLE 8 (comparatif)

Dans le même appareillage que celui utilisé pour l'Exemple 1 et dans les mêmes conditions que l'Exemple 7, à ceci près que l'on a introduit 0,2.10⁻³ mole de bis(di-t-butyl-2,4-phényl-6-phénoxy)-aluminium-isobutyle en solution dans l'ortho-xylène en lieu et place du bis(di-t-butyl-2,4-phényl-6-phénoxy)-magnésium, on a consommé 13,7 g d'éthylène en une heure de réaction. La composition des produits est donnée dans le Tableau 1. On recueille en outre 31,1 % en poids de polymère par rapport à l'éthylène consommé.

**TABLEAU 1**

| Exemple | Répartition des oligomères (% poids) | | | | Hexène-1 dans C6 (% poids) |
|---|---|---|---|---|---|
| | C4 | C6 | C8 | C10+ | |
| 1 | 1,3 | 84,4 | 1 | :2,3 | 98,9 |
| 2 | 1,1 | 82,7 | 1,1 | 2,4 | 99,7 |
| 3 | 1,7 | 70,2 | 2 | 3,9 | 98,4 |
| 4 | 20 | 5 | 0,5 | 2 | 55,0 |
| 5 | 0,6 | 85,4 | 0,7 | 2,4 | 99,4 |
| 6 | 2,3 | 74,2 | 0,6 | 2,4 | 95,2 |
| 7 | 1,7 | 70,5 | 1,6 | 3,5 | 98,0 |
| 8 | 2,6 | 61,1 | 1,1 | 4,1 | 97,3 |

## Revendications

1. Composition catalytique **caractérisée en ce qu'**elle comprend un mélange non supporté :
- d'au moins un composé du chrome ;
- avec au moins un composé aryloxy d'un élément M choisi dans le groupe formé par le magnésium, le calcium, le strontium, le baryum, de formule générale M(RO)₂₋ₙXₙ dans laquelle RO est un radical aryloxy contenant de 6 à 80 atomes de carbone, X est un halogène ou un radical hydrocarbyle contenant de 1 à 30 atomes de carbone, et n est un nombre entier qui prend l'une des valeurs 0 et 1 ; et
- avec au moins un composé d'aluminium choisi dans le groupe formé par les tris(hydrocarbyl)aluminiums et les composés chlorés ou bromés d'hydrocarbylaluminium, répondant à la formule générale AIR'ₘY₃₋ₘ dans laquelle R' est un radical hydrocarbyle comprenant de 1 à 6 atomes de carbone, Y est un atome de chlore ou de brome et m est un nombre de 1 à 3, et les aluminoxanes.

2. Composition selon la revendication 1, **caractérisée en ce que** le composé du chrome comporte un ou plusieurs anions identiques ou différents choisis dans le groupe formé par les halogénures, carboxylates, acétylacétonates, les anions alcoxy et aryloxy.

3. Composition selon l'une des revendications 1 et 2, **caractérisée en ce que** dans le composé aryloxy de l'élément M de formule générale M(RO)₂₋ₙXₙ, le radical aryloxy RO a pour formule générale 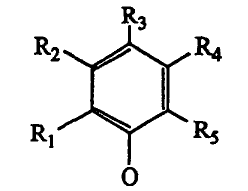 dans laquelle R₁, R₂, R₃, R₄, R₅, identiques ou différents, représentent un hydrogène, un halogène ou un radical hydrocarbyle comprenant de 1 à 16 atomes de carbone.

4. Composition selon l'une des revendications 1 à 3, **caractérisée en ce que** le composé aryloxy de l'élément M est le bis(2,6-diphénylphénoxy)-magnésium, le bis(2-tert-butyl-6-phénylphénoxy)-magnésium ou le bis(2,4-ditert-butyl-6-phénylphénoxy)magnésium.

5. Composition selon l'une des revendications 1 à 4, **caractérisée en ce que** le composé d'hydrocarbylaluminium est le dichloroéthylaluminium, le sesquichlorure d'éthylaluminium, le chlorodiéthylaluminium, le chlorodiisobutylaluminium, le triéthylaluminium, le tripropylaluminium, le triisobutylaluminium, le méthylaluminoxane.

6. Composition selon l'une des revendications 1 à 5, **caractérisée en ce que** le composé d' hydrocarbyl aluminium est le triéthylaluminium.

7. Composition selon l'une des revendications 1 à 6, **caractérisée en ce que** les composants du catalyseur sont mis en contact dans un solvant comprenant au moins un hydrocarbure saturé, au moins un hydrocarbure insaturé, oléfinique ou dioléfinique, et/ou au moins un hydrocarbure aromatique.

8. Composition selon l'une des revendications 1 à 7, **caractérisée en ce que** la concentration du chrome dans la solution catalytique est de 1.10⁻⁵ à 0,1 mole/L.

9. Composition selon l'une des revendications 1 à 8, **caractérisée en ce que** le rapport molaire entre le composé aryloxy de l'élément M et le composé du chrome est de 1:1 à 30:1 et le rapport molaire entre l'aluminium hydrocarbyle et le composé du chrome est de 1:1 à 35:1.

10. Procédé d'oligomérisation de l'éthylène en une composition comprenant principalement des hexènes, utilisant une composition catalytique selon l'une des revendications 1 à 9.

11. Procédé selon la revendications 10, **caractérisé en ce que** la réaction d'oligomérisation de l'éthylène est effectuée sous une pression de 0,5 à 15 MPa et à une température de 20 à 180 °C.

## Patentansprüche

1. Katalytische Zusammensetzung, **dadurch gekennzeichnet, dass** sie ein nicht getragenes Gemisch umfasst:
- von wenigstens einer Chromverbindung;
- mit wenigstens einer Alkoxyverbindung eines Elements M, das aus der durch Magnesium, Kalzium, Strontium, Barium gebildeten Gruppe gewählt ist, mit einer allgemeinen Formel M(RO)₂₋ₙXₙ, in der RO ein Aryloxyradikal ist, das 6 bis 80 Kohlenstoffatome enthält, X ein Halogen oder ein Kohlenwasserstoffrest mit 1 bis 30 Kohlenstoffatomen ist und n eine ganze Zahl ist, die einen der Werte 0 und 1 annimmt; und
- mit wenigstens einer Aluminiumverbindung, die aus der Gruppe gewählt ist, die gebildet wird durch die Tris(Kohlenwasserstoff)Aluminium und die chlorierten oder bromierten Aluminiumkohlenwasserstoffverbindungen, die der allgemeinen Formel AIR'ₘY₃₋ₘ entsprechen, in der R' ein Kohlenwasserstoffrest ist, der 1 bis 6 Kohlenstoffatome umfasst, Y ein Chlor- oder Bromatom und m eine Zahl von 1 bis 3 ist, und die Aluminoxane.

2. Zusammensetzung nach Anspruch 1, **dadurch gekennzeichnet, dass** die Chromverbindung eines oder mehrere identische oder verschiedene Anionen umfasst, die aus der Gruppe gewählt sind, die gebildet wird durch die Halogenide, Carboxylate, Acetylacetonate, Alkoxy- und Aryloxyanionen.

3. Zusammensetzung nach einem der Ansprüche 1 und 2, **dadurch gekennzeichnet, dass** in der Aryloxyverbindung des Elements M mit einer allgemeinen Formel M(RO)₂₋ₙXₙ das Aryloxyradikal RO als allgemeine Formel hat: 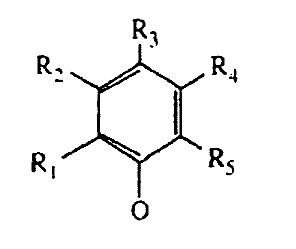 in der R₁, R₂, R₃, R₄, R₅ identisch oder verschieden ein Wasserstoff, ein Halogen, einen Kohlenwasserstoffrest, der 1 bis 16 Kohlenstoffatome umfasst, darstellen.

4. Zusammensetzung nach einem der Ansprüche 1 bis 3, **dadurch gekennzeichnet, dass** die Aryloxyverbindung des Elements M Bis(2,6-Diphenylphenoxy)-Magnesium, Bis(2-tert.-Butyl-6-Phenylphenoxy)-Magnesium oder Bis(2,4-Ditert.-Butyl-6-Phenylphenoxy-)Magnesium ist.

5. Zusammensetzung nach einem der Ansprüche 1 bis 4, **dadurch gekennzeichnet, dass** die Aluminiumkohlenwasserstoffverbindung Ethylaluminiumdichlorid, Ethylaluminiumsesquichlorid, Diethylaluminiumchlorid, Diisobutylaluminiumchlorid, Triethylaluminium, Tripropylaluminium, Triisobutylaluminium, Methylaluminoxan ist.

6. Zusammensetzung nach einem der Ansprüche 1 bis 5, **dadurch gekennzeichnet, dass** die Aluminiumkohlenwasserstoffverbindung Triethylaluminium ist.

7. Zusammensetzung nach einem der Ansprüche 1 bis 6, **dadurch gekennzeichnet, dass** die Katalysatorbestandteile in einem Lösungsmittel kontaktiert werden, das wenigstens einen gesättigten Kohlenwasserstoff, wenigstens einen ungesättigten, olefinischen oder diolefinischen Kohlenwasserstoff und/oder wenigstens einen aromatischen Kohlenwasserstoff umfasst.

8. Zusammensetzung nach einem der Ansprüche 1 bis 7, **dadurch gekennzeichnet, dass** die Chromkonzentration in der katalytischen Lösung 1·10⁻⁵ bis 0,1 mol/l ist.

9. Zusammensetzung nach einem der Ansprüche 1 bis 8, **dadurch gekennzeichnet, dass** das Molverhältnis zwischen der Aryloxyverbindung des Elements M und der Chromverbindung 1:1 bis 30:1 ist und das Molverhältnis zwischen dem Aluminiumkohlenwasserstoff und der Chromverbindung 1:1 bis 35:1 ist.

10. Verfahren zur Oligomerisierung von Ethylen zu einer Zusammensetzung, die hauptsächlich Hexene umfasst, indem eine katalytische Zusammensetzung nach einem der Ansprüche 1 bis 9 verwendet wird.

11. Verfahren nach Anspruch 10, **dadurch gekennzeichnet, dass** die Oligomerisierungsreaktion des Ethylens unter einem Druck von 0,5 bis 15 MPa und bei einer Temperatur von 20 bis 180°C durchgeführt wird.

## Claims

1. A catalytic composition **characterized in that** it comprises a non-supported mixture of:
- at least one chromium compound;
- with at least one aryloxy compound of an element M selected from the group formed by magnesium, calcium, strontium and barium, with general formula M(RO)₂₋ₙXₙ, where RO is an aryloxy radical containing 6 to 80 carbon atoms, X is a halogen or a hydrocarbyl radical containing 1 to 30 carbon atoms and n is a whole number that takes one of the values 0 and 1; and
- with at least one aluminium compound selected from the group formed by tris(hydrocarbyl)aluminium compounds and chlorinated or brominated hydrocarbylaluminium compounds, with general formula AIR'ₘY₃₋ₘ, where R' is a hydrocarbyl radical containing 1 to 6 carbon atoms, Y is a chlorine or bromine atom and m is a number from 1 to 3, and aluminoxanes.

2. A composition according to claim 1 **characterized in that** the chromium compound comprises one or more identical or different anions selected from the group formed by halides, carboxylates, acetylacetonates, and alkoxy or aryloxy anions.

3. A composition according to claim 1 or claim 2 **characterized in that** the aryloxy radical RO in the aryloxy compound of element M with general formula M(RO)₂₋ₙXₙ has general formula: 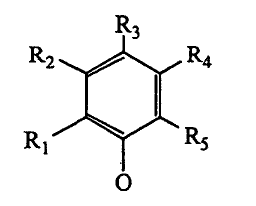 where R₁, R₂, R₃, R₄ and R₅, which may be identical or different, represent a hydrogen atom, a halogen atom or a hydrocarbyl radical containing 1 to 16 carbon atoms.

4. A composition according to any one of claims 1 to 3 **characterized in that** the aryloxy compound of element M is bis(2,6-diphenylphenoxy)magnesium, bis(2-tert-butyl-6-phenylphenoxy)magnesium or bis(2,4-di-tert-butyl-6-phenylphenoxy)-magnesium.

5. A composition according to any one of claims 1 to 4 **characterized in that** the hydrocarbylaluminium compound is dichloroethylaluminium, ethylaluminium sesquichloride, chlorodiethylaluminium, chlorodiisobutylaluminium, triethylaluminium, tripropylaluminium, triisobutylaluminium or methylaluminoxane.

6. A composition according to any one of claims 1 to 5 **characterized in that** the hydrocarbylaluminium compound is triethylaluminium.

7. A composition according to any one of claims 1 to 6 **characterized in that** the components of the catalyst are brought into contact in a solvent comprising at least one saturated hydrocarbon, at least one unsaturated olefinic or diolefinic hydrocarbon and/or at least one aromatic hydrocarbon.

8. A composition according to any one of claims 1 to 7 **characterized in that** the chromium concentration in the catalytic solution is in the range 1 x 10⁻⁵ to 0.1 mole/l.

9. A composition according to any one of claims 1 to 8 **characterized in that** the mole ratio between the aryloxy compound of element M and the chromium compound is 1:1 to 30:1, and the mole ratio between the hydrocarbylaluminium compound and the chromium compound is 1:1 to 35:1.

10. A process for the oligomerisation of ethylene to a composition mainly containing hexenes using a catalytic composition according to any one of claims 1 to 9.

11. A process according to claim 10 **characterized in that** the ethylene oligomerisation reaction is carried out at a pressure of 0.5 to 15 MPa and at a temperature of 20°C to 180°C.
